# EUROPÄISCHE PATENTANMELDUNG

(11) **EP 1 430 851 A2**
(43) Veröffentlichungstag der Anmeldung: **23.06.2004**
(21) Anmeldenummer: 03027991.3
(22) Anmeldetag: 05.12.2003
(51) Int. Cl.: A61C 1/18

(54) **Schnellkupplung zur Verbindung von Geräten eines medizinischen oder chirurgischen Handstücksystems mit einem Versorgungsschlauch**

(30) Priorität: 20.12.2002 AT 19102002
(71) Anmelder: W & H Dentalwerk Bürmoos GmbH, 5111 Bürmoos (AT)
(72) Erfinder: Kardeis, Richard, 5111 Bürmoos (AT); Wendtner, Wolfgang, 5112 Lamprechtshausen (AT)

(57) **Zusammenfassung**

Die Erfindung betrifft die Ausgestaltung der Schnittstelle zwischen Geräten eines medizinischen oder chirurgischen Handstücksystems und einem Versorgungsschlauch in Form einer Schnellkupplung sowie eine dafür besonders bevorzugte Ausführung der Schnellkupplung in Form einer formschlüssigen Steckverbindung mit einem Verriegelungselement und Verschlußelementen, z.B. Kugeln. Die Kupplung kann damit besonders kurz, leicht, sowie hygienisch und ergonomisch vorteilhaft ausgebildet sein.

## Beschreibung

Die Erfindung betrifft eine Schnellkupplung zur Verbindung von Geräten eines medizinischen oder chirurgischen Handstücksystems mit einem Versorgungsschlauch.

Medizinische Handstücksysteme, bestehend aus einem Behandlungsinstrument mit einer Vorrichtung zum Befestigen eines Behandlungswerkzeuges, einem Antriebsmotor, eventuell benötigten Zwischenstücken und Adaptern und einem Versorgungsschlauch, weisen mehrere Schnittstellen auf, die durch Kupplungen überbrückt werden. Gegenwärtig verwendet man dazu zwei Arten von Kupplungen:
Zwischen medizinischen Geräten, zum Beispiel einem Handstück als Behandlungsinstrument und einem Motor, werden verschiedene Arten von Schnellkupplungen, zum Beispiel Steck-Drehkupplungen, eingesetzt. Sie ermöglichen ein rasches Wechseln der Handstücke während einer Behandlung und ein umkompliziertes Lösen des Handstückes zur Sterilisation. Diese Schnellkupplungen werden meist durch kraftschlüssige Steckverbindungen realisiert, wobei die Übergabe verschiedener Medien, wie Wasser und Luft, über einen Kupplungszapfen erfolgt, an dessen Umfang die Medien austreten und dann im Gegenstück durch Ringkanäle weitergeführt werden, wodurch eine Drehbarkeit des Handstückes gegenüber dem Motor gegeben ist. Diese Kupplungen eignen sich besonders für Behandlungsinstrumente mit geringem Durchmesser aber relativ großen Baulängen bzw. Anwendungen bei denen eine große Baulänge keine Nachteile bedingt.
Die Verbindung zwischen dem Versorgungsschlauch und dem medizinischen Gerät, zum Beispiel einem Motor, ist gewöhnlich lösbar, aber nicht als Schnellkupplung ausgeführt, d.h. sie ist meist durch eine Verschraubung mit oder ohne Überwurfmutter oder gelegentlich durch Bajonettverschlüsse realisiert. Bisher waren Schnellkupplungen an dieser Stelle nicht nötig, da die Motoren nicht sterilisiert werden konnten und somit eine Lösbarkeit nur zum Tauschen eines kaputten Schlauches oder zum Service des Motors nötig war. Aufgrund der immer höher werdenden Anforderungen an die Hygiene und technischen Verbesserungen, die eine Sterilisation von Elektromotoren ermöglichen, besteht jedoch der dringliche Bedarf, auch an der Schnittstelle zwischen dem Versorgungsschlauch und einem medizinischen Gerät, bevorzugt einem Motor, eine für den Anwender schnell und unkompliziert zu handhabende, ergonomisch vorteilhafte, kostengünstige und einfach herzustellende Kupplung zu schaffen. Zusätzlich soll diese Kupplung die Baulänge des Systems möglichst wenig verlängern.
Zum Stand der Technik im Bereich der Schnittstelle zwischen einem Versorgungsschlauch und einem medizinischen Gerät, beispielsweise einem Handstück oder einem Motor, gehören Umbausysteme, wie z.B. aus der FR 2.709.658 B1 bekannt. Hierbei wird auf das kupplungsseitige Ende des Versorgungsschlauches ein Kupplungszwischenstück aufgeschraubt, ebenso auf das schlauchseitige Ende eines Instrumentes, diese beiden Zwischenstücke lassen sich dann über einen Bajonettverschluß kuppeln.
In der US 4,477,253 Patentschrift wird eine Kupplung beschrieben, die auf einem ringförmigen Segment mindestens eine Abflachung besitzt, die in das schlauchseitige Gegenstück paßt. Die Verriegelung erfolgt durch Verdrehen, die Fixierung durch einen Stift.

Nachteilig bei diesen beiden Systemen ist, daß beim Kuppeln bzw. Lösen eine Verdrehung des Handstücks gegenüber dem Versorgungsschlauch nötig ist, wodurch eine schnelle Kupplung und Entkupplung erschwert wird. Oftmals wird anstatt des Motors der Schlauch zumindest zum Teil verdreht, wodurch der Schlauch oder Teile davon abgenützt werden und brechen können. Auch wird erst beim Verdrehen die Verriegelung erreicht, dies bedeutet, daß bei nicht ordnungsgemäßer Montage die Gefahr besteht, daß Motor und Schlauch wieder getrennt werden bzw. keine Funktion eingehen können. Bei Umbausystemen (FR 2.709.658) sind zusätzliche Teile nötig, was die Kosten erhöht und mehr Schmutzkanten mit sich bringt, das System verlängert, sowie zusätzliches Gewicht bedeutet.
In der US 4,403,959 Patentschrift wird eine Schnellkupplung beschrieben, die eine Steck-Drehkupplung ist. Die Verriegelung erfolgt über Kugeln. Die Kugeln werden über eine U-förmige, rotationssymmetrische, federbeaufschlagte Hülse in Ausnehmungen einer weiteren Hülse gehalten, bis beim Zusammenschieben der beiden Kupplungsteile die Hülse zurückgeschoben wird und die Kugeln in Ausnehmungen auf dem Gegenstück einrasten. Beim Lösen der Kupplung wird eine Außenhülse zurückgezogen und gibt die Kugeln wieder frei.
Diese Steckkupplung entspricht den Kupplungen, die üblicherweise zwischen medizinischen Geräten, wie zum Beispiel einem Handstück und einem Motor verwendet werden. Diese als Drehkupplungen ausgeführten Kupplungen erweisen sich an der Schnittstelle Motor-Versorgungsschlauch jedoch als nachteilig, da alle Medienleitungen in einem Kupplungszapfen gefaßt werden müssen und damit eine erhöhte Baulänge und ein höheres Gewicht einhergehen, was wiederum den Schwerpunkt des gesamten Handstücksystems verändert und die Handhabung durch den Anwender erschwert.

Aufgabe der vorliegenden Erfindung ist es, eine Schnellkupplung zu schaffen, die für den Anwender schnell und einfach zu kuppeln und entkuppeln ist, sowie keine zusätzlichen Teile nötig macht, das Gewicht nicht erhöht und keine Verlängerung des Systems Handstück-Motor-Versorgungsschlauch bedingt. Die erfindungsgemäße Schnellkupplung soll bevorzugt an der Schnittstelle zwischen einem medizinischen Gerät, bevorzugt einem Motor, und dem Versorgungsschlauch einsetzbar sein.
Diese Aufgabe wird gemäß der vorliegenden Erfindung durch eine Schnellkupplung mit den Merkmalen des Anspruchs 1 gelöst. Vorteilhafte Weiterentwicklungen sind in den Unteransprüchen beschrieben.
Bei der erfindungsgemäßen Ausgestaltung nach Anspruch 1 weist die Schnellkupplung ein den Kupplungsvorgang auslösendes Verriegelungselement, bevorzugt eine Verriegelungsplatte, auf, die im Inneren eines der beiden Geräte angeordnet ist. Um die Kupplung auszulösen, muß der Anwender nur die beiden zu kuppelnden Geräte zusammenstecken. Die Verbindung ist formschlüssig und wird erst beim Zurückziehen der Kupplungshülse wieder gelöst. Durch die formschlüssige Verbindung können die Kupplung und der Motor mit einer glatten Oberfläche versehen sein, da nur relativ geringe Zugkräfte nötig sind um die Kupplung zu lösen. Damit kann die hygienische Anforderung Schmutzkanten zu vermeiden erfüllt werden. Das System ist kostengünstig, da keine zusätzlichen Zwischenstücke oder lange Kupplungszapfen zur Übergabe der Medien benötigt werden und aus dem selben Grund auch leicht und kurz, was für den Zahnarzt, der täglich mehrere Stunden mit dem Handstücksystem arbeitet, deutliche ergonomische Vorteile bedeutet. Ebenfalls durch die Vermeidung von Zwischenstücken entsteht nur eine Bruchlinie als Übergang zwischen Kupplung und Motor, was wiederum aus hygienischer Sicht wünschenswert, sowie für das optische Erscheinungsbild vorteilhaft ist.
Der vorliegenden Erfindung liegt weiters die Aufgabe zugrunde ein Handstücksystem zu schaffen, bei dem der an den Versorgungsschlauch anzuschließende Motor schnell, einfach und für den Versorgungsschlauch schonend ge- und entkuppelt werden kann. Diese Aufgabe wird gemäß der vorliegenden Erfindung durch ein Handstücksystem nach Anspruch 10 gelöst. Vorteilhafte Weiterentwicklungen sind in den Unteransprüchen beschrieben.
Bei der erfindungsgemäßen Ausgestaltung nach Anspruch 10 erfolgt die Verbindung mittels einer Schnellkupplung, bevorzugt einer formschlüssigen Steckverbindung, wobei der Kupplungsvorgang für den Anwender ausschließlich das Zusammenstecken des Versorgungsschlauchs mit dem Motor umfaßt.

Die Erfindung wird nachfolgend anhand eines bevorzugten Ausführungsbeispiels und Bezug nehmend auf die beigefügten Zeichnungen erläutert:
- Figur 1: zeigt einen Längsschnitt durch das erfindungsgemäße motorseitige Ende eines Versorgungsschlauches und das schlauchseitige Ende eines Motors im entkuppelten Zustand.
- Figur 2: zeigt einen Querschnitt durch die Kupplung an der Stelle des Verriegelungselementes.
- Figur 3: zeigt einen Elektromotor mit der erfindungsgemäßen motorseitigen Kupplungshälfte.
- Figur 4: zeigt einen Längsschnitt durch den Elektromotor und den Versorgungsschlauch in gekuppeltem Zustand.

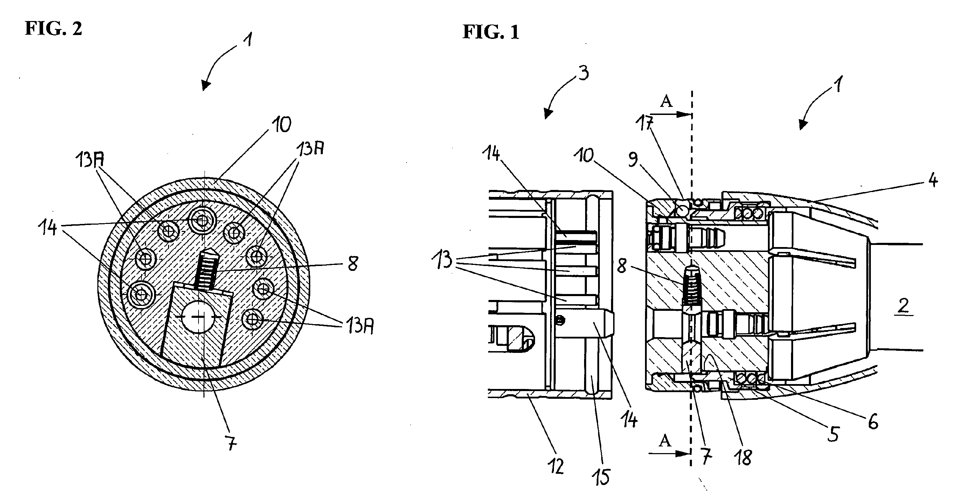

Figur 1 stellt das schlauchseitige Kupplungsstück 1, sowie das entsprechende Kupplungsstück des sterilisierbaren Motors 3 im entkuppelten Zustand dar. Die Schlauchhülse 2 dient zur Befestigung des flexiblen Schlauches, über den die benötigte Medien, wie Strom, Wasser und Luft, sowie ein Lichtleiter von der nicht dargestellten Dentaleinheit oder Steuerung zum Motor 3 gefördert werden. Die Außenhülse 4 des schlauchseitigen Kupplungsstückes 1 ist durch Verschrauben mit dem Ring 5 verbunden, der durch die Feder 6 beaufschlagt wird. Das im Inneren des schlauchseitige Kupplungsstücks 1 angeordnete Verriegelungselement 7 wird durch eine Verriegelungsfeder 8 vorgespannt und rastet in einen Absatz 18 des Ringes 5 ein, wodurch verhindert wird, daß sich die Feder 6 entspannt und wodurch der Ring 5 in einer in Richtung Schlauch (in der Figur 1 nach rechts) verschobenen Position fixiert wird. Das vordere Ende des Ringes 5 gibt damit eine Öffnung 17 in der Buchse 10 frei, in der mehrere Kugeln 9 versenkt sind.
Figur 2 stellt einen Schnitt entlang der Linie A-A durch das schlauchseitige Kupplungsstück 1 dar. Das Verriegelungselement 7, sowie die Verriegelungsfeder 8, die das Verriegelungselement 7 nach außen zur Buchse 10 drückt, sind hier abgebildet. Weiterhin sind die Elektrokontakte 13A, sowie die Medienleitungen 14 dargestellt.

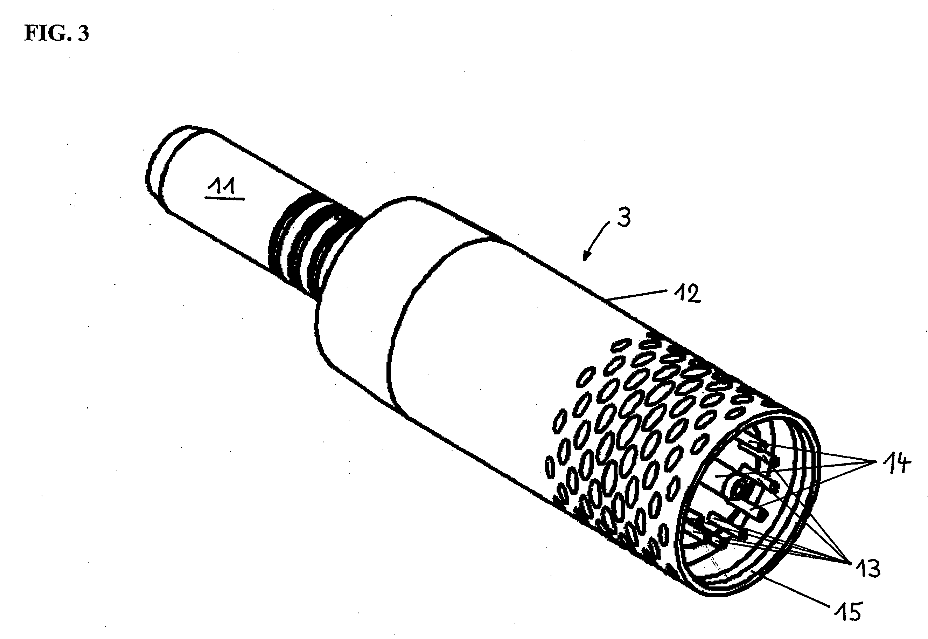

Figur 3 zeigt den Motor 3 in entkuppeltem Zustand. Auf den Kupplungszapfen 11 wird das nicht dargestellte Handstück mittels einer drehbaren Steckkupplung aufgesteckt. In der Motorhülse 12 befinden sich neben den anderen für die Funktion des Motors notwendigen Teilen Elektrokontakte 13 und Medienleitungen 14 für die Versorgung mit Luft, z.B. auch Kühlluft für den Motor und Wasser. Die Motorhülse 12 besitzt weiterhin eine ringförmige Nut 15, die zum Einrasten der Kugeln 9 als Verschlußelemente im gekuppelten Zustand dient.

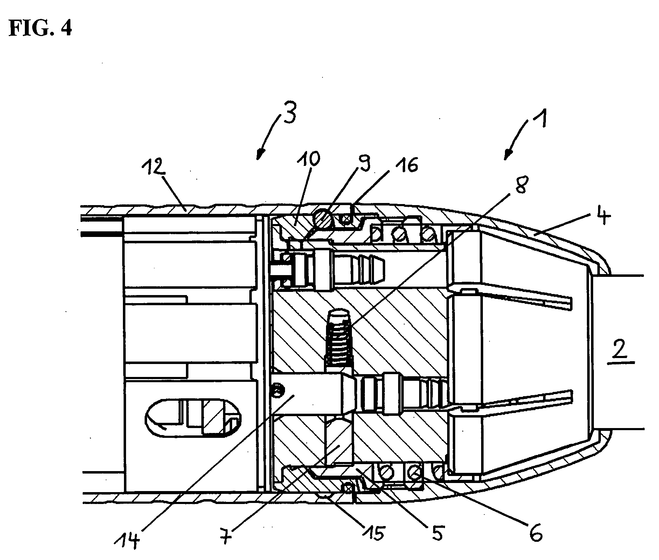

Figur 4 stellt den Motor 3 in gekuppeltem Zustand mit dem schlauchseitigen Kupplungsstück 1 dar. Der Übergang von der Außenhülse 4 des Kupplungsstückes 1 auf die Motorhülse 12 ist nur an der Bruchlinie 16 erkennbar. Beim Verbinden der beiden Kupplungsteile wird durch eine Medienleitung 14 (oder alternativ durch einen der Elektrokontake 13) des Motors 3 das Verriegelungselement 7 gegen seine Verriegelungsfeder 8 in das Innere des schlauchseitigen Kupplungsstückes 1 gedrückt, dadurch kann die Feder 6 entspannen und den Ring 5 in Richtung Motor 3 schieben, wodurch die Kugeln 9 nach außen in die Nut 15 des Motors gedrückt werden. Somit ist der Motor 3 mit dem Kupplungsstück 1 gekuppelt. Zum Entriegeln der Kupplung wird die Außenhülse 4 gegen die Feder 6 zurückgezogen, wodurch die Kugeln 9 wieder in die Öffnung 17 des Kupplungsstückes 1 wandern, dadurch löst sich der Motor 3 und die Leitung 14 gibt das Verriegelungselement 7 frei, das von der Verriegelungsfeder 8 nach außen gedrückt wird und somit den Ring 5 wieder blockiert.
Um beim Kuppeln ein sicheres Auslösen der Kupplung zu gewährleisten, ist zumindest jener Elektrokontakt (13) oder jene Medienleitung (14), die die Bohrung des Verriegelungselements (7) durchdringt, gleich lang wie die sie umgebende Außenhülse (12) oder überragt diese.

Die Erfindung ist nicht auf die dargestellten Ausführungsbeispiele beschränkt, sondern kann entsprechend der Bauweise des Motors oder des Versorgungsschlauches abgewandelt werden. So ist es insbesondere möglich anstatt Kugeln z.B. Rollen oder jedes andere zweckdienliche Element beliebiger Form zu verwenden. Die Ausführung des Verriegelungselementes kann ebenfalls verschiedentlich gestaltet sein. Es ist z.B. auch möglich anstatt von Verriegelungsfeder und -element nur eine Feder zu benutzen, die die Funktion beider Elemente erfüllt. Je nach den zu übertragenden Medien, kann das Anschlussbild des Motors anders aussehen und somit auch das Kupplungsstück anders ausgestaltet sein, dadurch kann es auch nötig werden, daß das Verriegelungselement an einer anderen Stelle oder in einer anderen Ausgestaltung verwendet wird.

## Patentansprüche

1. Formschlüssige Steckverbindung zur Schnellkupplung zweier Geräte (1, 3) eines medizinischen Handstücksystems, **dadurch gekennzeichnet, daß**
das den Kupplungsvorgang auslösende Verriegelungselement, bevorzugt eine Verriegelungsplatte (7), im Inneren eines der beiden Geräte (1, 3) angeordnet ist.

2. Formschlüssige Steckverbindung nach Anspruch 1, **dadurch gekennzeichnet, daß**
das Verriegelungselement (7) durch eine Feder (8) vorgespannt ist.

3. Formschlüssige Steckverbindung nach Anspruch 1 oder 2, **dadurch gekennzeichnet, daß**
das Verriegelungselement (7) eine Bohrung zum Durchführen einer Medienleitung (14) oder eines Elektrokontakts (13) aufweist.

4. Formschlüssige Steckverbindung nach Anspruch 3, **dadurch gekennzeichnet, daß** zumindest jener Elektrokontakt (13) oder jene Medienleitung (14), die die Bohrung des Verriegelungselements (7) durchdringt, gleich lang wie die sie umgebende Außenhülse (12) ist oder diese überragt.

5. Formschlüssige Steckverbindung nach einem der vorhergehenden Ansprüche, **dadurch gekennzeichnet, daß**
das Verriegelungselement (7) im entkuppelten Zustand in einem Absatz (18) des Rings (5) einrastet.

6. Formschlüssige Steckverbindung nach Anspruch 5, **dadurch gekennzeichnet, daß** der Ring (5) über eine Feder (6) vorgespannt ist.

7. Formschlüssige Steckverbindung nach Anspruch 6, **dadurch gekennzeichnet, daß** der Ring (5) mit einer Außenhülse (4) zum Entkuppeln der Steckverbindung verbunden ist.

8. Formschlüssige Steckverbindung nach einem der vorhergehenden Ansprüche, **dadurch gekennzeichnet, daß**
mehrere Medienleitungen (14) und/oder Elektrokontakte (13) ringförmig am äußeren Rand des Geräts (1, 3) angeordnet sind.

9. Formschlüssige Steckverbindung nach einem der vorhergehenden Ansprüche, **dadurch gekennzeichnet, daß**
im gekuppelten Zustand die Medienleitungen (14) und/oder die Elektrokontakte (13) im schlauchseitigen Kupplungsstück (1) mit jenen im Motor (3) fluchten.

10. Medizinisches Handstücksystem mit einem Elektromotor (3) zum Antrieb eines Behandlungsinstruments und einem Versorgungsschlauch (2), **dadurch gekennzeichnet, daß**
die Verbindung zwischen dem Elektromotor (3) und dem Versorgungsschlauch (2) mittels Schnellkupplung erfolgt.

11. Medizinisches Handstücksystem mit einem Motor (3) zum Antrieb eines Behandlungsinstruments und einem Versorgungsschlauch (2), **gekennzeichnet durch** eine Verbindung zwischen dem Motor (3) und dem Versorgungsschlauch (2) mittels Schnellkupplung, wobei der Kupplungsvorgang für den Anwender ausschließlich das Zusammenstecken des Versorgungsschlauchs (2) mit dem Motor (3) umfaßt.

12. Medizinisches Handstücksystem nach Anspruch 10 oder 11, **dadurch gekennzeichnet, daß**
die Schnellkupplung eine formschlüssige Steckverbindung ist.

13. Medizinisches Handstücksystem nach Anspruch 10, 11 oder 12, **dadurch gekennzeichnet, daß**
der Motor (3) ein Elektromotor, bevorzugt ein sterilisierbarer Elektromotor ist.

14. Medizinisches Handstücksystem nach einem der Ansprüche 10 bis 13, **gekennzeichnet durch**
eine formschlüssige Steckverbindung gemäß einem der Ansprüche 1 - 9.
